# EUROPEAN PATENT APPLICATION

(11) **EP 1 903 117 A1**
(43) Date of publication of application: **26.03.2008**
(21) Application number: 06450135.6
(22) Date of filing: 22.09.2006
(51) Int. Cl.: C12Q 1/68

(54) **Methods for the detection of mutations by means of primers that hybridize contiguously**

(71) Applicant: Veterinärmedizinische Universität Wien, 1210 Vienna (AT)
(72) Inventor: Czerny, Thomas, 1220 Vienna (AT)
(74) Representative: Sonn & Partner Patentanwälte

(57) **Abstract**

The present invention relates to methods and kits for the detection of mutations, in particular point mutations, in a nucleic acid molecule by means of primers that hybridize contiguously.

SEQUENCE LISTING

<110> Veterinärmedizinische Universität Wien

<120> Methods for the Detection of Mutations

<130> R 46638

<140> EP 06450135.6
<141> 2006-09-22

<160> 10

<170> PatentIn version 3.3

<210> 1
<211> 30
<212> DNA
<213> Artificial

<220>
<223> Primer

<400> 1
aagcaaagac acagaaacga aaagcagaga 30

<210> 2
<211> 10
<212> DNA
<213> Artificial

<220>
<223> Primer

<400> 2
tctctgcttt 10

<210> 3
<211> 10
<212> DNA
<213> Artificial

<220>
<223> Primer

<400> 3
tcgtttctgt 10

<210> 4
<211> 10
<212> DNA
<213> Artificial

<220>
<223> Primer

<400> 4
tcgtctctgt 10

<210> 5
<211> 10
<212> DNA
<213> Artificial

<220>
<223> Primer

<400>  5
gtctttgctt                                                              10


<210>  6
<211>  20
<212>  DNA
<213>  Artificial

<220>
<223>  Primer

<400>  6
tgagagcgaa tgtcgtagct                                                   20


<210>  7
<211>  21
<212>  DNA
<213>  Artificial

<220>
<223>  Primer

<400>  7
gagtgcacgc taaaggagag t                                                 21


<210>  8
<211>  21
<212>  DNA
<213>  Artificial

<220>
<223>  Primer

<400>  8
gcagctatca accagatcct g                                                 21


<210>  9
<211>  17
<212>  DNA
<213>  Artificial

<220>
<223>  Primer


<220>
<221>  misc_feature
<222>  (17)..(17)
<223>  dideoxycytosine

<400>  9
ggcagaagat ggcacgc                                                      17


<210>  10
<211>  20
<212>  DNA
<213>  Artificial

<220>
<223>  Primer

<400>  10
cataattgtc tcgcgctgac                                                   20

## Description

The present invention relates to methods for the detection of the presence of a first nucleic acid molecule in a sample wherein said first nucleic acid molecule has at least one sequence variation compared to a second nucleic acid molecule.

Sequence variations of DNA molecules are responsible, for instance, for the genetic diversity between organisms as well as for genetic disorders. The detection and determination of sequence variations is important for studying these phenomena, for the detection of alleles and for diagnosing genetic disorders.

Single-nucleotide polymorphisms (SNPs) are the most common individual variations, which are found every 250-350 bp and are responsible for the majority of genetic variation between human beings. Genetic variations (mutations or polymorphisms) result from DNA mutations that may or may not have functional consequences. Approximately 20% of DNA polymorphisms are length polymorphisms, and the remaining 80% are SNPs. Some SNPs may also be involved in disease processes. In addition, accurate and high throughput detection of genetic mutations in other species such as viruses, bacteria, fungi, plants and animals and further in artificial DNA constructs, like plasmids, is also highly desirable. Viral and bacterial mutations, for instance, play important roles in infection, drug sensitivity, drug resistance and escaping the immunological response generated to vaccines. The detection of mutations in artificial DNA constructs like plasmids and vectors, which may be employed in clinical practice, enables the skilled person to detect, for example, undesired mutations, which may negatively influence the therapeutic use of such constructs.

The detection of mutations in DNA is therefore of significant importance in a variety of fields, particularly with regard to diagnostic and prognostic applications. The diagnosis of genetically determined diseases and identification of disease carriers, for example, relies in large part on the identification of DNA mutations associated with the disease in individuals. Conventional methods involve the isolation of DNA, followed by subjecting said DNA to restriction endonucleases recognizing and cutting different restriction sites, gel electrophoresis and Southern blot analysis. In a further development Conner et al.(PNAS USA, 80 (1983): 278-282) suggest a method that does not require the mutation to be located within a restriction enzyme cleavage site. Said method involves the use of a labelled oligonucleotide probe which is able to hybridize to the region of the DNA which includes the possible mutation. The hybridization conditions are empirically determined to optimize differential hybridization of the probe to a nucleic acid sequence with the mutation, as opposed to a nucleic acid sequence lacking the mutation. An alternative method for the detection of a mutation within a DNA sequence involves a nucleic acid chip, which is covered with a number of DNA derived oligonucleotides. Said oligonucleotides selectively hybridize with the DNA to be analysed and based on detection signals obtained in comparison to the detected signals obtained by DNA without any mutations. Other methods for detecting mutations are SSCP (Single Strand Conformation Polymorphism), DGGE (Denaturing Gradient Gel Electrophoresis), DHPLC (Denaturing High Performance Liquid Chromatography) and mismatch cleavage.

Substantially all methods for the detection of sequence variations (e.g. mutations, in particular point mutations) known in the art are either time-consuming, labor intensive, not sufficiently sensitive, limited in their applicability, not universally applicable and/or expensive.

It is an object of the present invention to provide methods and means for the fast and simple detection of sequence variations between two or more nucleic acid molecules. These methods shall enable the detection of alleles, point mutations or SNPs which may be responsible for genetic disorders, splice variants etc. in a reliable and fast manner.

Therefore, the present invention relates to a method for the detection of the presence of a first nucleic acid molecule in a sample wherein said first nucleic acid molecule has at least one sequence variation compared to a second nucleic acid molecule comprising the steps of:
- providing a plurality of primers, wherein said primers hybridize contiguously to at least one stretch of said second nucleic acid molecule or to said second nucleic acid molecule covering the full length of said nucleic acid molecule and wherein said primers do not hybridize contiguously to said first nucleic acid molecule because said first nucleic acid molecule contains a sequence variation compared to said second nucleic acid molecule,
- providing a sample potentially comprising said first nucleic acid molecule,
- contacting said primers with said sample,
- adding at least one nucleic acid polymerase which incorporates nucleotides upon incubation for a sufficient time and at a sufficient temperature if the primers are not contiguously hybridized to the molecule, and
- detecting a nucleic acid polymerase activity indicating whether said incorporation has been performed, whereby the detection of said activity indicates the presence of said first nucleic acid molecules having at least one sequence variation. in the sample.

Template dependant DNA polymerases require for their activity single DNA strands as templates and at least one primer (an oligonucleotide comprising at least 5 nucleotides) in order to synthesize a second strand which is complementary to the template. If the template is covered entirely and contiguously with primers or with a complementary strand no DNA polymerase-catalyzed polymerization can occur, blocking the DNA polymerase activity and consequently no second strand complementary to the template is synthesized. If, however, one of the primers employed cannot bind or not efficiently bind to the template DNA, the DNA polymerase can synthesize a complementary strand at least in those regions of the DNA template which are not covered by a primer. The binding of a primer to the template DNA may be prevented or reduced in its efficiency when the first nucleic acid molecule (i.e. DNA template) compared to the second nucleic acid molecule and the complementary primer sequence shows a sequence variation.

As used herein, the term "sequence variation" refers to any single or multiple nucleotide substitutions, deletions, duplications, inversions or insertions in a first nucleic acid molecule compared to a second nucleic acid molecule. These nucleotide variations may be mutant or polymorphic allele variations. Furthermore also encompassed in this definition are splice variants of nucleic acid molecules.

The primers used in the method according to the present invention hybridize to contiguous portions of the first nucleic acid molecule (i.e. DNA template). Contiguous portions are zero (i.e., exactly contiguous so there is no space between the portions) nucleotides apart.

The primers (blocking primers) employed in a method according to the present invention hybridize contiguously to a nucleic acid molecule covering a stretch or the full length of said nucleic acid molecule, i.e. the blocking primers correspond to fragments of the second nucleic acid molecule. Therefore, if the second nucleic acid molecule comprises 1 to N nucleotides the plurality of blocking primers used herein hybridizes contiguously to the second nucleic acid molecules. The plurality of blocking primers can also be seen as fragments of the complementary strand of the second nucleic acid. Each primer can potentially initiate a DNA polymerse reaction, but is blocked in this reaction by the next contiguously binding primer. If however one blocking primer does not hybridize to the template molecule, a gap results and DNA synthesis catalyzed by a DNA polymerase occurs. The first (5') primer of the stretch of contiguously hybridising primers can be positioned somewhere within the template, further upstream of this primer no polymerase activity can therefore be initiated. All subsequent primers have to cover the template contiguously, but the stretch of blocking primers can be terminated by a modified primer. This modification has to be at the 3' end of the primer and has to be designed in a way that the polymerase can not initiate synthesis of the second strand from this primer (e.g. by adding a dideoxy-nucleotide). Therefore, the stretch of blocking primers can cover only part of the template nucleic acid molecules, but has to consist of contiguously hybridising primers, limited at its 3' end by a modified terminating primer. However mispriming of one of the primers at a position outside this stretch can potentially initiate polymerase activity unspecifically and therefore increase the background activity. Therefore short templates reduce potential mispriming and ideally the blocking primers cover the full length of the template molecules.

In order to allow the synthesis of a complementary strand, the polymerase, template and primer mixture have to be subjected to an appropriate temperature at which the polymerase is able to synthesize said strand if a sequence variation is present in the DNA template.

The temperature profile during the DNA synthesis of the method according to the present invention has to be designed in order to allow all the primers to hybridize specifically to the template DNA. Therefore it is preferred that the primers used exhibit similar melting temperatures (± 10°C, preferably ± 5°C, more preferably ± 2°C). The melting temperature of the primers may be optimised by varying the prior length (substantially without losing their specificities) and/or by using modified nucleotides (e.g. LNA nucleotides). Furthermore the reaction mixture may further be optimised by varying the concentration of the primers or by the addition of substances like DMSO (dimethyl sulfoxide), betaine, formamide and TMAC (tetramethylammonium chloride).

The primers according to the present invention can be obtained by simple chemical synthesis (e.g. Caruthers, M.H. (1983) Tetrahedron Lett. 24:245-248) or by enzymatic or chemical cleavage of a nucleic acid sequence comprising said primer sequences (e.g. second nucleic acid molecule).

"Hybridizing", as used herein, is the ability of primers to bind specifically to a nucleic acid sequence under stringent conditions and under conditions applied in the course of a polymerase chain reaction. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridisation with Nucleic Probes, "Overview of principles of hybridisation and the strategy of nucleic acid assays" (1993). Generally, stringent conditions are selected to be about 5 to 10°C lower than the thermal melting point (Tm) for the specific sequence at a defined ionic strength and pH. The Tm is the temperature (under defined ionic strength, pH, and nucleic acid concentration) at which 50% of the probes complementary to the target hybridize to the target sequence at equilibrium. The stringency further depends on the ionic strength and the pH, which for the presented method are limited by the reaction conditions of the polymerase.

The nucleic acid polymerase activity may be determined by various methods, whereby the most preferred methods require one single detection cycle (i.e. direct detection of the polymerase activity by using e.g. incorporation of modified nucleotides which then can be detected specifically in the product or the formation of byproducts of the reaction like inorganic pyrophosphate) or by repeated detection cycles thus creating nucleic acid fragments (e.g. PCR fragments).

According to a preferred embodiment of the present invention the first nucleic acid molecule is an amplification product of a nucleic acid amplification reaction, in particular of a polymerase chain reaction, a genomic fragment or a plasmid.

The method according to the present invention is especially suited for detecting sequence variations between two nucleic acid molecules with a limited number of nucleotides, like an amplification product of a nucleic acid amplification reaction (e.g. PCR, RT-PCR) or a plasmid. Of course it is also possible to detect sequence variations of fragments of high molecular nucleic acids (e.g. genomic DNA, chromosomal DNA). Furthermore, also cDNA or amplification products obtained by reverse transcription from e.g. mRNA may be used in a method according to the present invention.

The sequence variation is preferably a substitution, deletion or addition of at least one nucleotide, in particular single nucleotide polymorphisms and point mutations.

According to the present invention it is possible to detect any kind of sequence variation of a first nucleic acid compared to a second nucleic acid provided that the plurality of primers are not able to hybridize contiguously to the first nucleic acid in the course of a method according to the present invention. However, substitutions, deletions or insertions of one or more nucleotides or nucleic acid stretches are preferably detected.

According to a preferred embodiment of the present invention the primers consist of 5 to 60, preferably 7 to 40, more preferably 8 to 30, nucleotides.

Although the length of the primers to be used in a method according to the present invention can vary, the primers preferably comprise between 5 and 60 nucleotides. The length of the primers - among other factors (e.g. salt concentration, nucleotide composition) influences the annealing and melting temperature in the course of a nucleic acid polymerase reaction. Short primers (e.g. 10 to 18 nucleotides) can be used, for instance, at low temperatures and are therefore suitably used in connection with polymerases like the Klenow fragment. Furthermore, the binding of a short primer to a DNA template is influenced at a much higher degree by mismatches (e.g. sequence variations of one nucleotide) than long primers comprising e.g. 40 to 60 nucleotides. This will lead to a much better signal to noise ratio of short primers. In contrast thereto, long primers (exhibiting a reduced signal to noise ratio) may be suitably used for temperature stable DNA polymerases (e.g. Pfu, Pwo, Taq) and for sequence variations affecting longer stretches of the template nucleic acid molecules. If the primer length is too long (more than 60 nucleotides) the binding becomes more and more unspecific and consequently sequence variations, particularly in the middle of the primer, can hardly be identified.

Since not only the length of the primers exhibits an influence on the hybridization accuracy also other parameters, like the G+C content, primer concentration and salt concentration in the mixture have to be considered when suitable primers to be used in a method according to the present invention are selected.

At least one primer of said plurality of primers comprises preferably at least one modified nucleotide.

Modified nucleotides in primers may influence their hybridization properties and melting temperatures.

The modified nucleotide is preferably an LNA nucleotide and the primer containing said LNA nucleotide may be denominated LNA primer or oligonucleotide. The primer or primers may comprise at least one, two, three, four, five or more of said nucleotides.

LNA nucleotides are bicyclic nucleotides where a ribonucleoside is linked between the 2'-oxygen and the 4'-carbon atoms with a methylene unit (see e.g. EP 1 013 661). Locked Nucleic Acids (LNA) are a class of conformationally restricted oligonucleotide analogues. The main mechanism for LNA oligos binding plasmid DNA was demonstrated to be by strand displacement. LNA oligos can bind to DNA in a sequence-specific manner so that binding does not interfere with DNA conformation or gene expression. LNA oligonucleotides exhibit unprecedented thermal stabilities towards complementary DNA and RNA, which allow excellent mismatch discrimination. The high binding affinity of LNA oligos allows the use of short probes in antisense protocols and LNA may be used in any hybridization assay that requires high specificity and/or reproducibility, e.g., dual labelled probes, in situ hybridization probes, molecular beacons and PCR primers. Furthermore, LNA offers the possibility to adjust Tm values of primers and probes in multiplex assays. Each LNA base addition in an oligo increases the Tm by approximately 8°C. The synthesis and incorporation of LNA bases can be achieved by using standard DNA synthesis chemistry.

The primers to be used in a method according to the present invention may comprise at least one of said LNA bases in order to approximate the melting temperatures of the primers in order to provide a primer mixture having similar melting temperatures when contacting the DNA template. In addition these modifications can be useful to adjust the melting temperature of the primers with the ideal temperature of the polymerase. Furthermore application of LNA bases reduces the length of the primers for a given reaction temperature and thus increases the effect of single mismatches within the target sequence on the binding of the primer. The temperature of LNA primers and probes may be calculated by methods known in the art (e.g. Tolstrup N, et al. (2003) Nucleic Acids Res. 31:3758-62; McTigue PM. (2004) Biochemistry. 43:5388-405).

According to a preferred embodiment of the present invention the primers hybridize to the nucleic acid molecule of the sample under hybridization conditions at a temperature between 20 and 72°C, preferably between 25 and 70°C, more preferably between 30 and 68°C.

The primers used in a method according to the present invention may hybridize to the first nucleic acid molecule in a temperature range as outlined above. Especially short primers comprising e.g. 10 to 14 nucleotide residues for instance in combination with Klenow Fragment may already be employed at temperatures around 20 to 40°C.

The nucleic acid polymerase to be used in a method according to the present invention is preferably a template dependent DNA polymerase.

The present invention also refers to a method in which the primers hybridised to the first nucleic acid molecule are ligated together with a DNA ligase, preferably with a thermally stable ligase before the detection of the polymerase activity. After this ligation reaction the concatenated primers are considerably longer than the individual primers. Therefore a switch to a higher hybridisation temperature can be performed, which is significantly above the melting temperature for single primers. At these higher temperatures a mispriming of single primers is impossible and the background activity of the polymerase is largely reduced. The ligases to be used are preferably those regularly employed in ligase chain reactions (LCR).

According to a preferred embodiment of the present invention the polymerase activity is detected by determining the formation of an amplification product and/or by determining the incorporation of a modified nucleotide and/or by determining the release of a biochemical byproduct of the polymerase reaction, in particular inorganic pyrophosphate.

According to a preferred embodiment of the present invention an amplification product is obtained by adding a single reverse primer to the plurality of blocking primers. This reverse primer is preferably positioned downstream (3') of the terminating primer in order to prevent direct hybridisation with the blocking primers. In a first cycle the polymerase reaction may be initiated from a primer located directly 5' to a gap in the otherwise contiguously hybridising primers. The polymerase then synthesises a nucleic acid fragment covering the gap. Polymerases lacking 5'-3' exonuclease activity (e.g. Klenow Fragment, Pfu) will stop when they approach the next blocking primer, whereas polymerases containing such an activity (e.g. Taq) will digest all blocking primers positioned downstream of the gap and thus continue to synthesize the second strand to the end of the template. This extension product then serves as a template for the reverse primer, which is extended in the following cycle to the end of the previously synthesized fragment, demarcated by the last primer before the gap. The new fragment contains the same sequence variation as the template and therefore serves as an additional template for all subsequent reaction cycles. During these cycles, the number of synthesized fragments grows exponentially as in a polymerase chain reaction. The length of the resulting amplification product indicates the position of the sequence variation and is preferably determined by gel electrophoresis, capillary electrophoresis or HPLC. Alternatively the reaction is monitored by application of a real time polymerase chain reaction.

The application of a real time PCR in a method according to the present invention is advantageous because it allows a fast detection of the amplification. The nucleotide primers used in the real time PCR are preferably labelled with appropriate dyes regularly used in the art. Preferred dyes are fluorescent dyes. Furthermore the primers may comprise in addition to a dye a quencher (e.g. FAM/TAMRA). Real time PCR is preferably used because it allows to monitor continuously the potential amplification process (e.g. also by detecting the formation of a nucleic acid molecule with an intercalating agent like SYBR green) and, thus, the nucleic acid polymerase activity. The dye-labelled primers are preferably positioned between the terminating primer (3' to this primer) and the reverse primer.

According to a preferred embodiment of the present invention the reaction is conducted in the presence of at least one modified nucleotide.

The nucleotides which can potentially be incorporated into the product may comprise at least one modification. This modification may comprise, for instance, a substance which can bind to a binding partner so that a potentially produced product can be detected by the binding of said product comprising modified nucleotides to said binding partner. Furthermore also modified nucleotides may be employed which may be detected by their fluorescence or by a colour reaction after incorporation into an nucleic acid molecule.

The incorporation of modified nucleotides can be performed in a single polymerase reaction cycle or during the formation of an amplified product. For amplified products, a 5'-3' exonuclease activity of the polymerase is essential, whereas for single reaction cycles all DNA polymerases are applicable.

The nucleotide is preferably modified with a molecule selected from the group consisting of biotin, digoxigenin, fluorescein, guanosine, aminoderivatives, in particular aminoallyl, or fluorescent dyes.

According to another preferred embodiment of the present invention the performance of DNA synthesis is determined by detecting the release of inorganic pyrophosphate in the course of the nucleotide incorporation.

Synthesis of a nucleic acid product by a nucleic acid polymerase, preferably a DNA polymerase, is accompanied by the release of pyrophosphate from a deoxynucleotide triphosphate, which will be incorporated as diphosphates into the nucleic acid. The pyrophosphate formed in the course of a nucleic acid amplification can be determined by assays involving e.g. pyrophosphatase or other enzymes.

The release of inorganic pyrophosphate is preferably detected by using ATP sulphurylase and luciferase.

ATP sulfurylase quantitatively converts pyrophosphate to ATP in the presence of adenosine 5' phosphosulfate. The ATP formed in this reaction drives the luciferase-mediated conversion of luciferin to oxyluciferin that generates visible light in amounts that are proportional to the amount of ATP. The light produced in the luciferase-catalyzed reaction is detected e.g. by a charge coupled device (CCD) camera or photomultiplier.

According to a preferred embodiment of the present invention different nucleotides (nucleotide triphosphates) are added either to separate aliquots of sample-primer mixture or successively to the sample-primer mixture and subjected to the polymerase reaction to indicate which nucleotide is incorporated.

The determination of the polymerase activity by the detection of pyrophosphate release allows further to sequence the amplified product (e.g. Ahmadian A et al. Clin Chim Acta. (2006) 363:83-94, electronically published on 13 September 2005). This is especially advantageous when a sequence variation is detected in a nucleic acid molecule according to the present invention and in the same reaction mixture said sequence variation of the fragment is sequenced. This allows to directly identify the kind of the sequence variation (e.g. point mutations or SNPs). Since each incorporation event is accompanied by release of pyrophosphate, in a quantity equimolar to the amount of incorporated nucleotide, it is possible to determine the type of said nucleotide as well as the amount of said nucleotide incorporated into the product. Application of a polymerase lacking 5'-3' exonuclease activity will limit the sequencing reaction to the gap containing the sequence variation, whereas polymerases containing this activity will continue the reaction to the end of the template by removing the downstream blocking primers. Contrary to the incorporation of modified nucleotides, this application has to be performed in a single detection cycle.

The nucleotides added to the nucleic acid polymerasereaction are preferably degraded.

Where ATP is present in the reaction mixture during or after chain extension, for example as an impurity or as a contaminant of dATP added as the source of the base to be incorporated, it will also interfere in the pyrophosphate luciferin system and give an incorrect luminescence reading. Furthermore it is also crucial to remove the other nucleotides in the amplification mixture because these nucleotides may be incorporated in the amplified nucleic acid by forming pyrophosphate and consequently interfere with the sequencing steps. In order to continuously degrade unincorporated dNTPs and excess ATP, respective means have to be added to the reaction mixture. After degradation of the nucleotides, another dNTP is added (see e.g. US 6,210,891).

According to a preferred embodiment of the present invention the nucleotides are degraded by a nucleotide degrading enzyme, in particular by apyrase.

Apyrase is a calcium-activated enzyme that catalyzes the hydrolysis of ATP to yield AMP and orthophosphate. It can also act on ADP and other nucleoside triphosphates and diphosphates. Any unincorporated nucleotides in the reaction mixture are degraded by apyrase, before the next nucleotide is added to the tube and the cascade of reactions repeated (see e.g. US 6,210,891).

According to a preferred embodiment of the present invention the sequence variation is preferably a single nucleotide polymorphism or a point mutation.

The method according to the present invention is especially suitably employed in the detection of point mutations. Such mutations may have no effect on the health or may be responsible for inherited genetic defects causing various diseases or for somatic mutations leading e.g. to cancer. Therefore it is important to have suitable methods in order to detect point mutations in a quick and accurate manner.

Yet another aspect of the present invention relates to the use of a method according to the present invention for the detection of sequence variations in therapeutic plasmids.

Therapeutic plasmids or other therapeutic nucleic acids, which may be administered to a human or animal body, have to fulfil several criteria before they are released on the market. One of the major criteria is the lack of sequence variations compared to the originating nucleic acid. Therefore the method and the kit according the present invention may be used as a quality control for therapeutic nucleic acids.

Another aspect of the present invention relates to a kit for the detection of at least one sequence variation in a first nucleic acid molecule compared to a second nucleic acid molecule comprising at least two of the components:
- a plurality of primers, wherein said primers hybridize contiguously to a stretch of said second nucleic acid molecule or to said second nucleic acid molecule and wherein said primers do not hybridize contiguously to said first nucleic acid molecule, if said first nucleic acid molecule contains a sequence variation compared to said second nucleic acid molecule, and
- a nucleic acid polymerase, and/or
- an enzyme selected from the group consisting of ATP sulphurylase, luciferase and apyrase, and/or
- a detection means for determining the presence of a product of the polymerase reaction, in particular antibodies, Streptavidin, and/or
- a detection means for determining the production of an amplified nucleic acid molecule, in particular SYBR green, TaqMan probes, and/or
- a positive control being a sequence variant of the second nucleic acid molecule, and/or
- a negative control being the second nucleic acid molecule.

The kit according to the present invention comprising at least two of the above identified components may be used to perform any method disclosed herein.

The present invention is further illustrated by the following figures and examples without being restricted thereto.

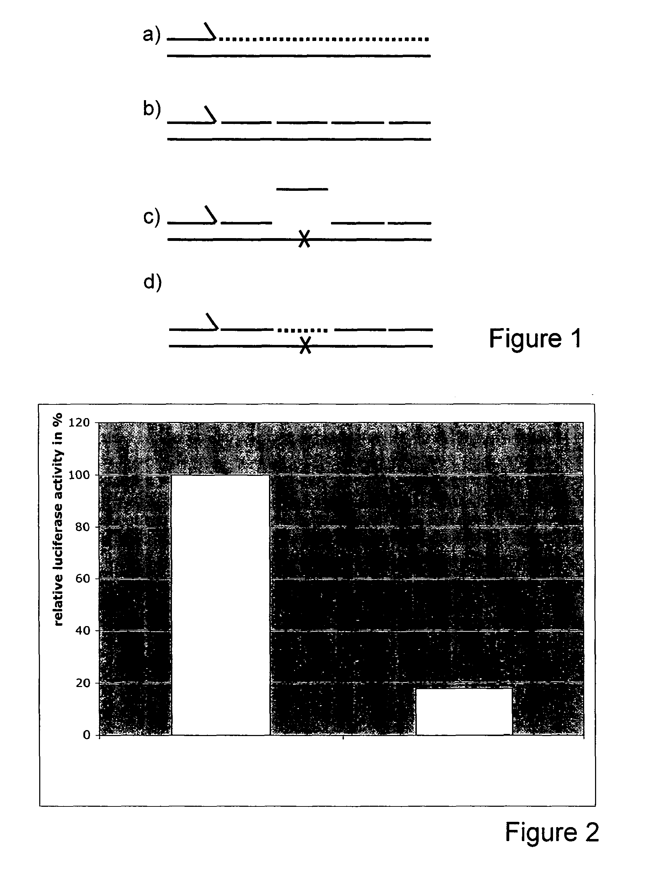

Fig. 1 shows the principle of the mutation detection method and a schematic presentation of the method. Full polymerase activity (dotted line in a) is blocked by binding of individual short oligos, covering the whole template (b). In case of a mutation the respective oligo can not bind (c) and therefore polymerase activity can be detected (d).

Fig. 2 shows the inhibition of polymerase activity by application of a blocking-primer. Single stranded DNA template was incubated either with primer (2) (left part) or with primers (2) and (3) (right part). In the reaction mixture was DNA polymerase and nucleotides and components to detect polymerase activity by light emission (luciferase activity). Polymerase activity was clearly down regulated by addition of the blocking primer (3).

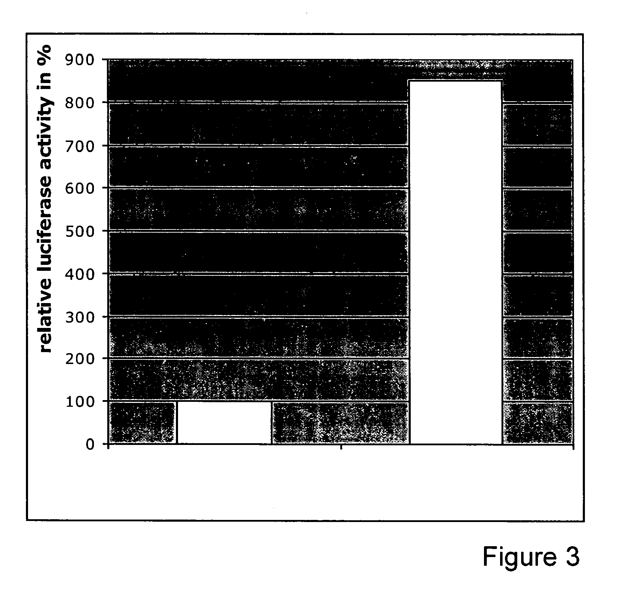

Fig. 3 shows the release of polymerase activity by mutation in the blocking-primer. Single stranded DNA template was completely covered with primers (1), (2) and (3) (left part) resulting in low activity. Use of a mutated primer (2mut) instead of (2) led to dramatic increase in polymerase activity (right part). For reaction conditions see examples and Fig. 1.

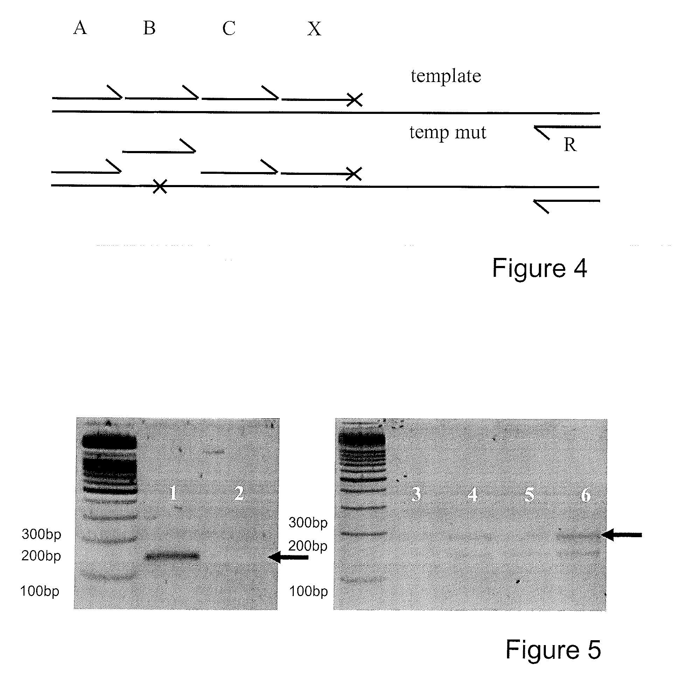

Fig. 4 shows the mutation detection method resulting in a PCR product. Schematic presentation of the primers and the template DNA used for Examples 3 and 4. (A), (B) and (C) are normal primers, (X) is a terminating primer containing a dideoxy-nucleotide at its 3' end. (R) is the reverse primer for the PCR reaction. (template) is wild-type template DNA, (temp mut) contains a point mutation.

Fig. 5 shows the results for Example 3 and 4. PCR was performed with the following primers: lane (1), primers (C) and (R); lane 2, primers (C), (X) and (R); lane (3) to (6), primers (A), (B), (C), (X) and (R). Template DNA was wild-type [lanes (1), (2), (3) and (5)], or mutated [lanes (4) and (6)]. Annealing temperatures were 53°C [lanes (1), (2), (3) and (4)], or 55°C [lanes (5) and (6)]. Position of the critical PCR fragment is indicated by an arrow. Blocking by primer (X) results in loss of the PCR fragment in (2) compared to (1). PCR fragments appear for mutated template DNA (4 and 6), but not with wild-type DNA (3 and 5).

### Example 1:

To provide a proof of principle for the described method the following experiments (Example 1 and 2) were performed. Polymerase activity in these experiments was quantitatively measured by luminometric inorganic pyrophosphate detection as described in Pal Nyren et al. Analytical Biochemistry (1993) 208, 171-175.

The luminescence was measured by a standard luminometer. Final reaction volume was 100 µl. To a mixture of 50 µl, additional 50 µl water containing the nucleotides were added, mixed and the tube was then immediately inserted into the luminometer and the measurement started with a delay of exactly 5 seconds (measuring time 10 seconds).

The mixture contained: 200 mM Tris pH 7.75, 4 mM EDTA, 0.1 % BSA, 10 mM MgCl₂, 1 mM DTT, 0.4 mg/ml PVP (360 000), 100 µg/ml D-luciferin, 2.1 µg/ml adenosine 5'-phosphosulfate, 0.3 U/ml sulfurylase, 0.5 U/ml Klenow fragment and 10 µg luciferase. To this mixture at room temperature the template DNA strand and the primers were added and the reaction started by adding 50 µl of the nucleotides dGTP, dCTP and dTTP each at 10 µM. As template a single stranded oligonucleotide was selected. The sequence of the template does not contain T in order to avoid problems with the addition of dATP (Ronaghi et al. Analytical Biochemistry [1996] 242, 84-89). Sequences of the template oligonucleotide was 5' -AAGCAAAGACACAGAAACGAAAAGCAGAGA-3' (SEQ ID No. 1), primer (1) 5'-TCTCTGCTTT-3' (SEQ ID No. 2), primer (2) 5'-TCGTTTCTGT-3' (SEQ ID No. 3), primer (2mut) 5'-TCGTCTCTGT-3' (SEQ ID No. 4) and primer (3) 5'-GTCTTTGCTT-3' (SEQ ID No. 5).

In the first experiment, the potential of a blocking-primer to inhibit further extension was tested. For this purpose primer (2) was added at a concentration of 10 pmol/100µl to the template (1 pmol/100µl) and the resulting luciferase signal measured. Next, the blocking-primer (3) was added at 10 pmol/100µl. In this reaction the production of light was dramatically reduced, demonstrating the blocking potential of perfectly fitting primers (see left and right part of Fig. 2, respectively).

### Example 2:

In the next experiment, the difference in blocking efficiency between a perfectly fitting primer and a primer containing one mismatch was detected. For this purpose the template was first incubated with primers (1), (2) and (3). The activity of the polymerase in this blocked situation served as a basal level. Next, the middle primer (2) was replaced by a primer containing a single mismatch (2mis) at exactly the same concentration, resulting in clearly activated polymerase activity (see Fig. 3; template concentration 1 pmole/100µl and all primers at 10 pmole/100µl).

### Example 3:

Using repeated cycles of polymerase activity for the presented method results in the formation of a PCR fragment, which is detectable according to its size. This principle was applied to example 3 and 4. The primers used for these experiments were (in 5'-3' orientation): (A), TGAGAGCGAATGTCGTAGCT (SEQ ID No. 6); (B), GAGTGCACGCTAAAGGAGAGT (SEQ ID No. 7); (C), GCAGCTATCAACCAG-ATCCTG (SEQ ID No. 8); (X), GGCAGAAGATGGCACG-dideoxyC (SEQ ID No. 9) and (R), CATAATTGTCTCGCGCTGAC (SEQ ID No. 10) (see Fig. 4 for positions of the primers within the template DNA). Primers (A), (B), (C) and (X) hybridize contiguously to the template, the reverse primer (R) is positioned in a distance to (X). Template DNA (temp) for the reactions was a plasmid containing a fragment of the mouse Lef1 gene (length of the PCR product (A)-(R) 180 bp). A mutated version of this gene (temp mut) contained a point mutation at the indicated position (see Fig. 4).

First the function of a blocking primer in a PCR reaction was tested. Primer (X) containing a dideoxy-nucleotide at its 3' terminus was used to block primer (C) in a reaction with (R). In standard PCR reactions, the detection of blocking activity of primer (X) failed. At temperatures above the annealing temperature the primers first loose their blocking potential, whereas initiation of elongation by the polymerase is still possible. In a standard PCR reaction this happens during the ramp between the annealing temperature and the 72°C elongation temperature, where Taq polymerase reaches full activity. Due to the short length of the PCR product, even complete elimination of the elongation step resulted in the formation of a PCR product, indicating that the temperature ramp alone is already sufficient for the polymerase to finish elongation of the fragment. Therefore the amount of Taq polymerase was limited, which indeed prevented the formation of a PCR product under these conditions. When the time of the annealing step was stepwise increased, the formation of a PCR product was observed, but this time the peak activity of Taq polymerase was shifted to the annealing step. Therefore conditions where the polymerase activity is restricted to a defined low temperature were generated. Here the primers have optimal conditions for annealing and blocking of the reaction worked efficiently (see Fig. 5).

The optimized conditions for blocking were finally found for a 20 µl reaction at: 10ng DNA template; 20 pmol of primers (C), (X) and (R); 0.01 units Taq polymerase; 20 cycles of 30 seconds denaturation (94°C) and 60 minutes (annealing + polymerase reaction) at 53°C. Other parameters like Mg²⁺ and nucleotides were used according to standard PCR reactions.

### Example 4:

With optimized conditions for blocking, the detection of a point mutation was tested next. For this purpose all 5 primers of Example 3 were mixed and applied in parallel to wild-type and mutated template DNA. The conditions finally leading to optimal detection efficiency (difference between the signal for the wild-type sequence and the mutated DNA) were for a 20 µl reaction: 10ng DNA template; 20 pmol for all primers; 0.03 units Taq polymerase, 10 cycles of 30 seconds denaturation and 10 minutes (annealing + polymerase reaction) at 53°C. The results are presented in Figure 5. The formation of a 180 bp fragment is only seen for mutated template DNA (reactions 4 and 6), but not for the wild-type version (reactions 3 and 5). A weak fragment was observed in all reactions at 160 bp, originating from incomplete blockage of primer (X). Fine tuned optimization of the primer concentration and/or application of modified primers eliminated this fragment.

## Claims

1. Method for the detection of the presence of a first nucleic acid molecule in a sample wherein said first nucleic acid molecule has at least one sequence variation compared to a second nucleic acid molecule comprising the steps of:
- providing a plurality of primers, wherein said primers hybridize contiguously to at least one stretch of said second nucleic acid molecule or to said second nucleic acid molecule covering the full length of said nucleic acid molecule and wherein said primers do not hybridize contiguously to said first nucleic acid molecule because said first nucleic acid molecule contains a sequence variation compared to said second nucleic acid molecule,
- providing a sample potentially comprising said first nucleic acid molecule,
- contacting said primers with said sample,
- adding at least one nucleic acid polymerase which incorporates nucleotides upon incubation for a sufficient time and at a sufficient temperature if the primers are not contiguously hybridized to the molecule, and
- detecting a nucleic acid polymerase activity indicating whether said incorporation has been performed, whereby the detection of said activity indicates the presence of said first nucleic acid molecules having at least one sequence variation in the sample.

2. Method according to claim 1, **characterized in that** said first nucleic acid molecule is an amplification product of a nucleic acid amplification reaction, in particular of a polymerase chain reaction, genomic DNA, cDNA or a plasmid.

3. Method according to claim 1 or 2, **characterized in that** the sequence variation is a substitution, deletion or addition of at least one nucleotide, in particular single nucleotide polymorphisms and point mutations.

4. Method according to any one of claims 1 to 3, **characterized in that** the primers consist of 5 to 60, preferably 7 to 50, more preferably 8 to 30, nucleotides.

5. Method according to any one of claims 1 to 4, **characterized in that** at least one primer of said plurality of primers comprises at least one modified nucleotide.

6. Method according to claim 5, **characterized in that** the modified nucleotide is an LNA nucleotide.

7. Method according to any one of claims 1 to 6, **characterized in that** the primers hybridize to the nucleic acid molecule of the sample under hybridization conditions at a temperature between 20 and 72°C, preferably between 25 and 70°C, more preferably between 30 and 68°C.

8. Method according to any one of claims 1 to 7, **characterized in that** the primers hybridized to the first nucleic acid molecule are ligated with a DNA ligase, preferably with a thermally stable ligase.

9. Method according to any one of claims 1 to 8, **characterized in that** the polymerase activity is detected by determining the formation of an amplification product and/or by determining the incorporation of a modified nucleotide and/or by determining the release of a byproduct of the reaction such as inorganic pyrophosphate.

10. Method according to claim 9, **characterized in that** the amplification is a polymerase chain reaction and a polymerase containing 5'-3' exonuclease activity is used.

11. Method according to claim 9 or 10, **characterized in that** the amplification product is determined by a method selected from the group consisting of gel electrophoresis, capillary electrophoresis and HPLC.

12. Method according to claim 11, **characterized in that** the polymerase chain reaction is monitored by means of a real time polymerase chain reaction.

13. Method according to any one of claims 9 to 12, **characterized in that** the nucleic acid amplification is conducted in the presence of at least one modified nucleotide.

14. Method according to claim 9, **characterized in that** the nucleotide is modified with a molecule selected from the group consisting of biotin, digoxigenin, guanosine, aminoderivatives, in particular aminoallyl, and fluorescent dyes.

15. Method according to claim 9, **characterized in that** the release of inorganic pyrophosphate is detected by using ATP sulphurylase and luciferase.

16. Method according to claim 9 or 15, **characterized in that** different nucleotides are added either to separate aliquots of sample-primer mixture or successively to the sample-primer mixture and subjected to the polymerase reaction to indicate which nucleotide is incorporated.

17. Method according to claim 16, **characterized in that** the nucleotides added to the nucleic acid amplification reaction are degraded.

18. Method according to claim 17, **characterized in that** the nucleotides are degraded by a nucleotide degrading enzyme, in particular by apyrase.

19. Use of a method according to any one of claims 1 to 18 for the detection of single nucleotide polymorphisms and point mutations of a first nucleic acid molecule compared to a second nucleic acid molecule.

20. Use of a method according to any one of claims 1 to 18 for the detection of sequence variations in therapeutic plasmids.

21. Kit for the detection of the presence of a first nucleic acid molecule in a sample wherein said first nucleic acid molecule has at least one sequence variation compared to a second nucleic acid molecule comprising at least two of the components:
- a plurality of primers, wherein said primers hybridize contiguously to a stretch of said second nucleic acid molecule or to said second nucleic acid molecule covering the full length of said nucleic acid molecule and wherein said primers do not hybridize contiguously to said first nucleic acid molecule because said first nucleic acid molecule contains a sequence variation compared to said second nucleic acid molecule, and
- a nucleic acid polymerase, and/or
- an enzyme selected from the group consisting of ATP sulphurylase, luciferase and apyrase, and/or
- a detection means for determining the presence of a product of the polymerase reaction, in particular antibodies, Streptavidin, and/or - a detection means for determining the production of an amplified nucleic acid molecule, in particular SYBR green, TaqMan probes, and/or
- a positive control being a sequence variant of the second nucleic acid molecule, and/or
- a negative control being the second nucleic acid molecule.
